# EUROPEAN PATENT SPECIFICATION

(11) **EP 0 494 239 B1**
(45) Date of publication and mention of the grant of the patent: **20.12.1995**
(21) Application number: 90915158.1
(22) Date of filing: 24.09.1990
(51) Int. Cl.: G01N 33/44, B29C 39/44

(54) **RESIN MOULDING APPARATUS AND METHODS**
VERFAHREN UND VORRICHTUNG ZUM FORMEN VON KUNSTSTOFFEN
PROCEDE ET APPAREIL POUR LE MOULAGE DE RESINES

(30) Priority: 22.09.1989 GB 8921481
(43) Date of publication of application: 15.07.1992
(73) Proprietor: FORD-WERKE AKTIENGESELLSCHAFT, 50725 Köln (DE); FORD MOTOR COMPANY, Dearborn, MI 48120 (US); FORD FRANCE S. A., 92506 Rueil-Malmaison Cédex (FR); FORD MOTOR COMPANY LIMITED, Brentwood, Essex CM13 3BW (GB)
(72) Inventor: HUTCHEON, Keith, Finer, Beeston Nottingham NG9 2TH (GB)
(74) Representative: Messulam, Alec Moses
(86) International application number: GB9001469
(87) International publication number: WO9104487

(56) References cited:
- GB-A- 2 227 567
- US-A- 3 791 792
- Patent Abstracts of Japan, vol., 7, no. 256, (P-236)(1401), 15 November 1983, & JP, A, 58139057 (MITSUBISHI DENKI K.K.) 18 August 1983.
- Patent Abstracts of Japan, vol., 10, no. 127 (P455)(2184), 13 May 1986, & JP, A, 60252251 (KOGYO GIJUTSUIN (JAPAN) 12 December 1985.
- Patent Abstracts of Japan, vol., 9, no. 190 (P-378)(1913), 7 August 1985, & JP, A, 6058541 (KOGY GIJUTSUIN (JAPAN) ) 4 April 1985.
- Patent Abstracts of Japan, vol., 10, no. 86 (P-443)(2143), 4 April 1986, & JP, A, 60224052 (KOGY FIJUTSUIN (JAPAN) ) 8 November 1985.
- Patent Abstracts of Japan, vol., 8, no. 94 (M-293)(1581), 28 April 1984, & JP, A, 599015 (MITSUBISHI DENKI K. K. ) 18 January 1984.

## Description

This invention relates to resin moulding apparatus and methods.

The investigation and control of resin moulding processes often requires means for detecting the presence of resin in the mould, or its state of cure. For this purpose, thermocouples have been installed in resin moulding apparatus, the arrival of resin at the sensor, or the onset of the curing process, being indicated as a change in temperature by the thermocouple. Such thermocouples can be unreliable and give rise to spurious readings.

An alternative approach is disclosed in JP-A 58 139 057.

According to the abstract in Patent Abstracts of Japan, 7, 256, (P236) (1401) filling of a metal injection mould with resin is detected by a fall in pressure in an exhaust pipe connected to a cavity at the top of the mould when the resin reaches and fills this cavity. The progress of gelling and cooling is then measured by measuring the electrical properties of the resin in a cell formed by a cavity in a seal between the upper and lower parts of the mould, which are used as electrodes.

The present invention seeks to provide a resin moulding process in which the presence or condition of the resin in a mould can be detected more reliably than by use of thermocouples, and in which both the arrival and the progress of curing of the resin can be detected by the same means.

According to the invention, in a resin injection moulding process injection of the resin into a mould is controlled in response to a voltage signal generated electrolytically in the cell formed on contact of the injected resin with two electrodes of different metals. Suitably, a two-piece mould is used of which the upper half is of metal and the electrodes are this upper mould half and an electrically isolated insert of a different metal the surface of which defines part of the inner surface of this upper mould half.

The invention also includes resin moulding apparatus comprising a two-piece injection mould made up of upper and lower halves defining a mould cavity, the upper mould half being of metal, wherein part of the inner surface of the upper mould half comprises an electrode of a different metal, electrically isolated therefrom, and the electrode and upper mould half are electrically connected to means for detecting and monitoring the output of the electrolytic cell formed when injected resin contacts both the insert and the upper mould half.

The invention makes use of the discovery that when a resin used as moulding material is in a plastic state, in particular in an uncured or partially-cured state, the resin can behave as an electrolyte. As well as using the voltage generated by an electrolytic cell in which the resin acts as an electrolyte to detect and control the filling of the mould with resin, it may also be used to control the operation of the injection moulding apparatus during the curing stage.

With reference to earlier application GB-A 2 227 567, which is not part of the state of the art under Article 54 EPC, the applicant has voluntarily limited the scope of the present application and submitted separate claims for the United Kingdom.

Although it is not wished to limit the scope of the present invention by theory, it is believed that resins are capable of acting as electrolytes because they contain free radicals in the liquid, uncured or partially cured state.

The invention is therefore particularly applicable to injection moulding systems incorporating thermosetting resins, such as polyester and vinyl ester resins, optionally cross-linked with styrene, phenolic resins and epoxy resins. Other types of resin and specific examples thereof are well known to persons skilled in the art. Such resins are cured by the application of heat, and contain catalysts, accelerators, promotors or initiators, some or all of which are responsible for the creation of free radicals in the resin composition.

The invention may also be applicable to resin systems based on thermoplastic resins where the system results in free radicals in its plastic state and in which the resin itself is in a form suitable for injection moulding.

A preferred embodiment of the invention will now be described, by way of example only, with reference to the accompanying drawings, in which:
Figure 1 is a cross-section of an electrode assembly;
Figure 2 shows graphs of the temperature (T) in degrees centigrade and the EMF (V) in volts developed across the electrode assembly of Figure 1 against time (t) in seconds;
Figure 3 is a partial cross-section of resin moulding apparatus in accordance with the invention; and
Figure 4 is a graph of the electrical output of the apparatus of Figure 3.

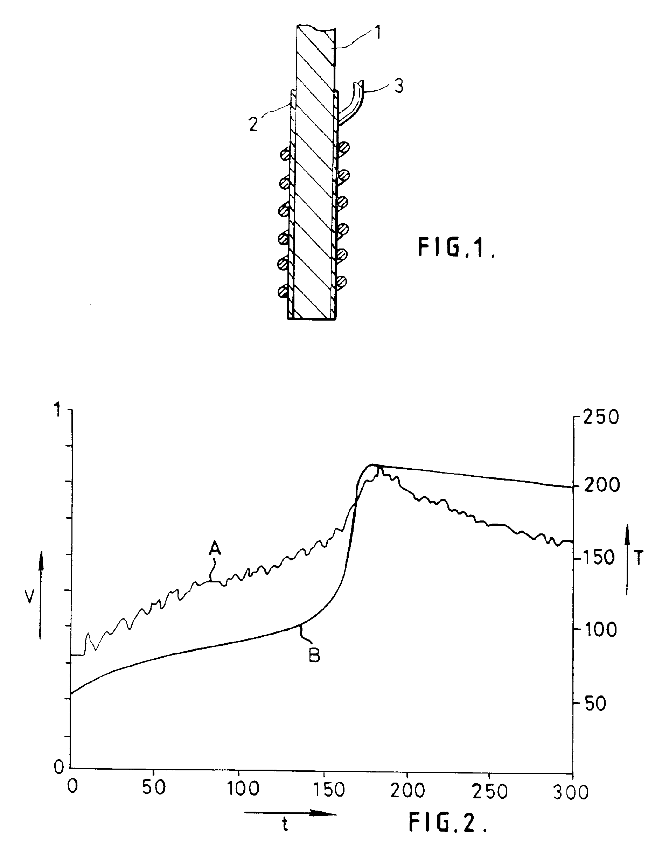

Referring to the drawings, Figure 1 shows an electrode assembly used to demonstrate the ability of molten resin to act as an electrolyte. The electrode assembly comprises an aluminium rod 1 covered with glass-fibre braid 2 and having a copper wire 3 wound helically around the rod over the braid. The glass braid keeps the two electrodes out of direct contact with each other. When the assembly is immersed in molten polyester resin, the resin can contact both electrodes.

Figure 2 is a graph illustrating by line A the voltage developed across the electrode assembly when immersed in a polyester resin in the process of gelling and curing. The temperature of the resin, as measured by a separate thermocouple, is indicated by line B. As can be seen, the voltage rises to a peak of about 900mv after about 200 seconds, corresponding to the peak exotherm of the resin during gelation. The voltage decreases thereafter as the resin cures and cools. The characteristics indicate that the electrolytic properties of the resin are capable of being used to detect the gelation of the resin.

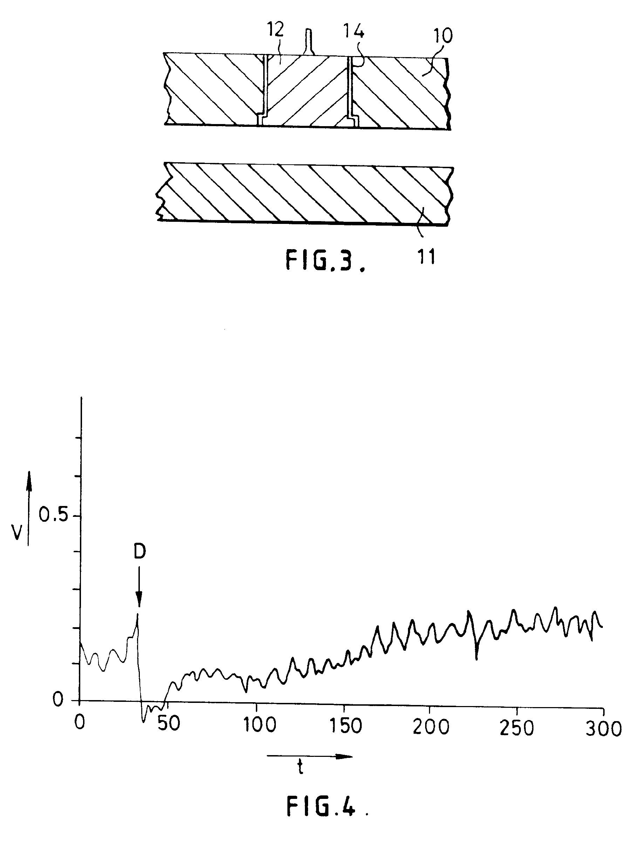

Figure 3 illustrates a two-piece mould suitable for producing specimens of plastics materials by resin transfer moulding. The mould has an upper mould half 10, composed of aluminium, that can be brought into sealed contact with a lower mould half 11 to define a mould cavity into which liquid plastics moulding material, such as polyester resin, may be injected.

A hole 14 is formed in the upper mould half at a position remote from the point at which resin is injected. A copper plug 12 is mounted in the hole 14 so that the lower face of the plug lies flush with the inner surface of the upper mould half. The plug 12 is secured in the hole 14 by means on an epoxy resin adhesive, which electrically insulates the plug 12 from the upper mould half. The upper mould half 10 and the plug 12 are connected by electrical leads to a high-impedance digital voltmeter. The inner surface of the upper mould half and the copper plug may be coated with a releasing agent prior to injection of resin in the mould.

Figure 4 is a graph against time of the voltage measured between the copper plug and the aluminium mould half as polyester resin at room temperature was injected into the mould, which had an initial temperature of 110°C. As indicated, although the voltage signal undergoes considerable variation due to "noise", the voltage between the plug and the upper mould half remains relatively steady during the first 40 seconds of the injection process, during which resin flows into the mould towards the copper plug 12. When the resin fills the mould to the level of the plug 12, the voltage between the plug and the upper mould half drops significantly, as indicated by the arrow D, indicating that whilst the resin is in a liquid, uncured or partially cured state, it acts as an electrolyte between the plug 12 and the upper mould half. Thereafter, the voltage increases gradually to a steady state, again subject to "noise ", indicating that the curing process is complete.

Figures 2 and 4 illustrate that resin is capable of acting as an electrolyte whilst in a plastic state, and that this capability may be used additionally to provide a means for detecting the extent to which resin has cured, or the presence of resin in a mould. Either signal may be used to control the operation of resin injection moulding equipment.

Although the above description refers only to copper and aluminium as electrode materials, it will be apparent to any person skilled in the art that a similar effect will be achieved provided that different metals are used for the two electrodes, and that the metals are otherwise compatible with the resin and are capable of withstanding the moulding conditions.

Similarly, although only polyester resins have been mentioned above, other moulding materials may be used.

## Claims (Claims for the following Contracting State(s): DE, ES, FR, IT, NL)

1. A resin injection moulding process wherein injection of the resin into a mould is controlled in response to a voltage signal generated electrolytically in the cell formed on contact of the injected resin with two electrodes of different metals.

2. A resin injection moulding process according to claim 1 wherein the mould is a two-piece mould (10, 11) of which the upper half is of metal and in which the electrodes are this upper mould half (10) and an insert (12) of a different metal forming part of the inner surface of this upper mould half and electrically isolated from direct contact therewith.

3. A resin injection moulding process according to claim 1 or claim 2 wherein the voltage signal is also used to control the operation of the process during curing of the resin.

4. Resin moulding apparatus comprising a two-piece injection mould made up of upper and lower halves (10, 11) defining a mould cavity, the upper mould half (10) being of metal, characterised in that part of the inner surface of the upper mould half comprises an electrode (12) of a different metal, electrically isolated from direct contact therewith, and that the electrode and upper mould half are electrically connected to means for detecting and monitoring the output of the electrolytic cell formed when injected resin contacts both the insert and the upper mould half.

5. Resin moulding apparatus according to claim 4, wherein said electrode (12) is in the form of a plug mounted in a hole in the upper mould half.

6. Resin moulding apparatus according to claim 4 or claim 5, wherein said electrode (12) is of copper and the upper mould half (10) is of aluminium.

## Claims (Claims for the following Contracting State(s): GB)

1. Resin moulding apparatus comprising a two-piece injection mould made up of upper and lower halves (10, 11) defining a mould cavity, the upper mould half (10) being of metal, characterised in that part of the inner surface of the upper mould half comprises an electrode (12) of a different metal, electrically isolated from direct contact therewith, and that the electrode and upper mould half are electrically connected to means of detecting and monitoring the output of the electrolytic cell formed when injected resin contacts both the insert and the upper mould half.

2. Resin moulding apparatus according to claim 1, wherein said electrode (12) is in the form of a plug mounted in a hole in the upper mould half.

3. Resin moulding apparatus according to claim 1 or claim 2, wherein said electrode (12) is of copper and the upper mould half (10) is of aluminium.

4. A resin injection moulding process wherein in a resin moulding apparatus according to any of claims 1 to 3 injection of the resin into the mould is controlled in response to a voltage signal generated electrolytically in the cell formed on contact of the injected resin with the electrode (12) and the upper mould half (10).

5. A resin injection moulding process according to claim 4 wherein the voltage signal is also used to control the operation of the process during curing of the resin.

## Patentansprüche (Patentansprüche für folgende(n) Vertragsstaat(en): DE, ES, FR, IT, NL)

1. Ein Kunststoff-Spritzgießverfahren, worin das Einspritzen des Harzes in eine Form als Antwort auf ein Spannungssignal gesteuert wird, das elektrolytisch in der Zelle erzeugt wird, die sich durch den Kontakt des eingespritzten Harzes mit zwei Elektroden aus unterschiedlichen Metallen bildet.

2. Ein Kunststoff-Spritzgießverfahren nach Anspruch 1, worin die Form eine zweiteilige Form (10, 11) ist, deren obere Hälfte aus Metall besteht, und in der die Elektroden diese obere Hälfte der Form (10) und ein Einsatz (12) aus einem anderen Metall sind, der einen Teil der inneren Oberfläche dieser oberen Hälfte der Form bildet und vom. direkten Kontakt zu dieser isoliert ist.

3. Ein Kunststoff-Spritzgießverfahren nach Anspruch 1 oder Anspruch 2, worin das Spannungssignal ebenso zum Steuern des Ablaufs des Verfahrens beim Aushärten des Harzes verwendet wird.

4. Eine Vorrichtung zum Formen von Kunststoffen, umfassend eine zweiteilige Spritzgießform, die aus einer einen Formenhohlraum definierenden oberen und unteren Hälfte (10, 11) aufgebaut ist, wobei die obere Hälfte (10) der Form aus Metall ist, dadurch gekennzeichnet, daß ein Teil der inneren Oberfläche der oberen Hälfte der Form eine Elektrode (12) aus einem anderen Metall umfaßt, die vom direkten Kontakt dazu isoliert ist, und daß die Elektrode und die obere Hälfte der Form elektrisch an einer Vorrichtung zum Erfassen und zum Überwachen der Abgabe der elektrolytischen Zelle angeschlossen sind, die sich bildet, wenn eingespritzter Kunststoff sowohl den Einsatz als auch die obere Hälfte der Form berührt.

5. Eine Vorrichtung zum Formen von Kunststoffen nach Anspruch 4, worin diese Elektrode (12) die Form eines in eine Öffnung in der oberen Hälfte der Form eingesetzten Stopfens besitzt.

6. Eine Vorrichtung zum Formen von Kunststoffen nach Anspruch 4 oder Anspruch 5, worin diese Elektrode (12) aus Kupfer und die obere Hälfte der Form (10) aus Aluminium sind.

## Patentansprüche (Patentansprüche für folgende(n) Vertragsstaat(en): GB)

1. Eine Vorrichtung zum Formen von Kunststoffen, umfassend eine zweiteilige Spritzgießform, die aus einer einen Formenhohlraum definierenden oberen und unteren Hälfte (10, 11) aufgebaut ist, wobei die obere Hälfte (10) der Form aus Metall ist, dadurch gekennzeichnet, daß ein Teil der inneren Oberfläche der oberen Hälfte der Form eine Elektrode (12) aus einem anderen Metall umfaßt, die vorm direkten Kontakt dazu isoliert ist, und daß die Elektrode und die obere Hälfte der Form elektrisch an einer Vorrichtung zum Erfassen und zum Überwachen der Abgabe der elektrolytischen Zelle angeschlossen sind, die sich bildet, wenn eingespritzter Kunststoff sowohl den Einsatz als auch die obere Hälfte der Form berührt.

2. Eine Vorrichtung zum Formen von Kunststoffen nach Anspruch 1, worin diese Elektrode (12) die Form eines in eine Öffnung in der oberen Hälfte der Form eingesetzten Stopfens besitzt.

3. Eine Vorrichtung zum Formen von Kunststoffen nach Anspruch 1 oder Anspruch 2, worin diese Elektrode (12) aus Kupfer und die obere Hälfte der Form (10) aus Aluminium sind.

4. Ein Kunststoff-Spritzgießverfahren, worin in einer Vorrichtung zum Formen von Kunststoffen nach irgendeinem der Ansprüche 1 bis 3 das Einspritzen des Harzes in die Form als Antwort auf ein Spannungssignal gesteuert wird, das elektrolytisch in der Zelle erzeugt wird, die sich aus dem Kontakt des eingespritzten Harzes mit der Elektrode (12) und der oberen Hälfte der Form (10) bildet.

5. Ein Kunststoff-Spritzgießverfahren nach Anspruch 4, worin das Spannungssignal ebenso zum Steuern des Ablaufs des Verfahrens beim Aushärten des Harzes verwendet wird.

## Revendications (Revendications pour l'(les) Etat(s) contractant(s) suivant(s): DE, ES, FR, IT, NL)

1. Procédé de moulage de résines par injection dans lequel l'injection de la résine dans un moule est commandée en réponse à un signal de tension généré de manière électrolytique dans la cellule formée lors du contact de la résine injectée avec deux électrodes constituées de métaux différents.

2. Procédé de moulage de résines par injection selon la revendication 1, dans lequel le moule est un moule en deux pièces (10, 11) dont la moitié supérieure est faite de métal et dans lequel les électrodes sont cette moitié supérieure de moule (10) et une pièce rapportée (12) d'un métal différent formant une partie de la surface intérieure de cette moitié supérieure de moule et isolé électriquement du contact avec celle-ci.

3. Procédé de moulage de résines par injection selon la revendication 1 ou la revendication 2, dans lequel le signal de tension est également utilisé pour commander le fonctionnement du procédé pendant la polymérisation de la résine.

4. Dispositif de moulage de résines comprenant un moule d'injection en deux pièces constitué de moitiés supérieure et inférieure (10, 11) définissant une cavité de moule, la moitié supérieure de moule (10) étant faite de métal, caractérisé en ce qu'une partie de la surface intérieure de la moitié supérieure de moule comprend une électrode (12) faite d'un métal différent, isolée électriquement du contact direct avec celle-ci, et en ce que l'électrode et la moitié supérieure de moule sont reliées électriquement à des moyens destinés à détecter et à surveiller la sortie de la cellule électrolytique formée lorsque de la résine injectée vient en contact avec à la fois la pièce rapportée et la moitié supérieure de moule.

5. Dispositif de moulage de résines selon la revendication 4, dans lequel ladite électrode (12) est sous la forme d'un bouchon monté dans un trou ménagé dans la moitié supérieure de moule.

6. Dispositif de moulage de résines selon la revendication 4 ou la revendication 5, dans lequel ladite électrode (12) est faite de cuivre et la moitié supérieure de moule (10) est faite d'aluminium.

## Revendications (Revendications pour l'(les) Etat(s) contractant(s) suivant(s): GB)

1. Dispositif de moulage de résines comprenant un moule d'injection en deux pièces constitué de moitiés supérieure et inférieure (10, 11) définissant une cavité de moule, la moitié supérieure de moule (10) étant constituée de métal, caractérisé en ce qu'une partie de la surface intérieure de la moitié supérieure de moule comprend une électrode (12) faite d'un métal différent, isolée électriquement du contact direct avec celle-ci, et en ce que l'électrode et la moitié supérieure de moule sont reliées électriquement à des moyens de détection et de surveillance de la sortie de la cellule électrolytique formée lorsque la résine injectée vient en contact avec à la fois la pièce rapportée et la moitié supérieure de moule.

2. Dispositif de moulage de résines selon la revendication 1, dans lequel ladite électrode (12) est sous la forme d'un bouchon monté dans un trou ménagé dans la moitié supérieure de moule.

3. Dispositif de moulage de résines selon la revendication 1 ou la revendication 2, dans lequel ladite électrode (12) est faite de cuivre et la moitié supérieure de moule (10) est faite d'aluminium.

4. Dispositif de moulage de résines par injection dans lequel dans un dispositif de moulage de résines selon l'une quelconque des revendications 1 à 3, l'injection de la résine dans le moule est commandée en réponse à un signal de tension généré de manière électrolytique dans la cellule formée lors du contact de la résine injectée avec l'électrode (12) et la moitié supérieure de moule (10).

5. Procédé de moulage de résines par injection selon la revendication 4, dans lequel le signal de tension est également utilisé pour commander le fonctionnement du procédé pendant la polymérisation de la résine.
